(19) Europäisches Patentamt
European Patent Office
Office européen des brevets

(11) Veröffentlichungsnummer: **0 219 042 B1**

# (12) EUROPÄISCHE PATENTSCHRIFT

(45) Veröffentlichungstag der Patentschrift: **02.08.89**

(21) Anmeldenummer: **86113875.8**

(22) Anmeldetag: **07.10.86**

(51) Int. Cl.⁴: **C07C 1/24**, C07C 11/12, C07C 11/18, B01J 29/28, B01J 29/18, B01J 29/04, C07C 1/253

(54) **Verfahren zur Herstellung von Dienen durch Dehydratisierung von Aldehyden.**

(30) Priorität: **15.10.85 DE 3536665**

(43) Veröffentlichungstag der Anmeldung: **22.04.87 Patentblatt 87/17**

(45) Bekanntmachung des Hinweises auf die Patenterteilung: **02.08.89 Patentblatt 89/31**

(84) Benannte Vertragsstaaten: **BE CH DE FR GB IT LI NL**

(56) Entgegenhaltungen:
**EP-A- 0 162 385**

**CHEMICAL ABSTRACTS, Band 103, 21. Oktober - 4. November 1985, Seite 651, Spalte 2, Zusammenfassungsnr. 141131a, Columbus, Ohio, US; J.B. NAGY et al.: "Reactivity of isopropanol on potassium-and cenium-exchanged ZSM-5 and mordenite"**

(73) Patentinhaber: **BASF Aktiengesellschaft, Carl-Bosch-Strasse 38, D-6700 Ludwigshafen(DE)**

(72) Erfinder: **Hoelderich, Wolfgang, Dr., Mannheimer Strasse 18 c, D-6710 Frankenthal(DE)**
Erfinder: **Hettinger, Peter, Dr., Schloss-Strasse 3, D-6803 Edingen-Neckarhausen(DE)**
Erfinder: **Merger, Franz, Dr., Max-Slevogt-Strasse 25, D-6710 Frankenthal(DE)**

EP 0 219 042 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft ein Verfahren zur Herstellung von Dienen durch Dehydratisierung von Aldehyden unter Verwendung von Zeolith-Katalysatoren, die mit Caesium dotiert oder die silaniert sind.

Diene sind wegen ihrer vielseitigen Verwendungsmöglichkeiten gesuchte chemische Verbindungen. Man benötigt sie z. B. in der Gummi- und Kunststoffindustrie sowie als Ausgangs- und Zwischenverbindungen bei mannigfaltigen organischen Reaktionen. Die Herstellung von Dienen aus Aldehyden durch einfache Dehydratation ist wünschenswert, da die Aldehyde z. B. über die Oxosynthese leicht zugänglich sind. Man führt sie z. B. an phosphorhaltigen Katalysatoren durch. Dabei werden als Katalysatoren z. B. Säuren, wie Phosphorsäure (DE-OS 2 163 396), Borphosphate (EP 80 449) und Ammoniumaluminiumsulfate (GB 2 063 297) verwendet. Bei diesen Verfahren ist die Wasserdampfverdünnung von Nachteil. Außerdem läßt sich eine Regenerierung der durch Koksabscheidung desaktivierten Katalysatoren nicht oder nur schwer durchführen.

Es wurde nun gefunden, daß man bei der katalytischen Dehydratisierung von Aldehyden zu Dienen bei höherer Temperatur besonders vorteilhafte Ergebnisse erzielt, wenn man die Dehydratisierung unter Verwendung von Zeolith-Katalysatoren vornimmt, die mit Caesium dotiert oder die silanisiert sind.

Mit den erfindungsgemäß zu verwendenden Katalysatoren werden bei hohem Umsatz und hoher Selektivität lange Standzeiten erzielt. Die lange Standzeit des Katalysators wird auch unter Ausschluß von Wasserdampf erhalten. Weiterhin ist es vorteilhaft, daß sich die erfindungsgemäß zu verwendenden Katalysatoren nach evtl. auftretender Desaktivierung durch Koksbildung leicht mit Luft regenerieren lassen.

Aldehyde, die sich nach dem erfindungsgemäßen Verfahren zu Dienen dehydratisieren lassen, sind z. B. Aldehyde der Formel

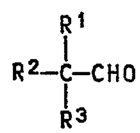 (I)

in der die Reste $R^1$, $R^2$ und $R^3$ gleich oder verschieden sind und jeweils einen aliphatischen, cycloaliphatischen, aromatischen oder heterocyclischen Rest mit 1 bis 10 C-Atomen bedeuten und $R^1$ und $R^2$ auch für Wasseratome stehen können.

Aliphatische Reste sind z.B. geradkettige oder verzweigte Alkylreste mit 1 bis 8 C-Atomen, cycloaliphatische Reste sind z.B. Cyclohexylreste und aromatische Reste sind z.B. Phenylreste.

Bevorzugt sind Aldehyde der Formel

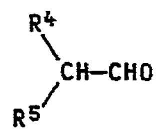 (II),

In der Formel II sind die Reste $R^4$ und $R^5$ gleich oder verschieden. $R^4$ bedeutet einen Alkylrest mit 1 bis 3 C-Atomen oder ein Wasserstoffatom und $R^5$ einen Alkylrest mit 1 bis 8 C-Atomen oder einen Kohlenwasserstoffrest, der zusammen mit $R^4$ einen Cyclohexylrest bildet.

Als Aldehyde der genannten Art sind insbesondere solche geeignet, die an einem alpha-ständigen C-Atom ein Wasserstoffatom tragen. Beispielsweise seien die folgenen Aldehyde genannt: Butanal, 2-Methylbutanal, 2-Methylpentanal, 2-Ethylhexanal, Pivalinaldehyd, 2-Benzylpropanal, 2-Ethylbutanal, Cyclohexylaldehyd und Isovaleraldehyd. Die Ausgangsverbindungen lassen sich beispielsweise durch Oxosynthese aus Olefinen herstellen. So wird z.B. 2-Methylbutanal durch Hydroformylierung von Buten-2 gewonnen.

Als Katalysatoren werden für die erfindungsgemäße Dehydratisierung der Aldehyde zu den Dienen Zeolithe eingesetzt, die mit Caesium dotiert oder die silaniert sind.

Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffions, ausgeglichen. Ein Kationenaustausch ist möglich. Die Räume zwischen den Tetraedern sind vor der Dehydration durch Trocknen bzw. Calcinieren von Wassermolekeln besitzt.

Entsprechend ihrer Struktur werden Zeolithe in verschiedene Gruppen unterteilt (s. Ullmanns Encyclopädie d. techn. Chemie, 4. Auflage, Bd. 24, Se. 575 (1983)). So bilden bei der Mordenit-Gruppe Ketten oder bei der Chabasit-Gruppe Schichten aus Tetraedern die Zeolith-Struktur, während sich bei der

Faujasit-Gruppe die Tetraeder zu Polyedern ordnen, z.B. in Form eines Kuboktaeders, der aus Vierringen bzw. Sechsringen aufgebaut ist. Je nach Verknüpfung der Kuboktaeder, wodurch unterschiedlich große Hohlräume und Poren entstehen, unterscheidet man Zeolithe vom Typ A, X oder Y.

Nach dem erfindungsgemäßen Verfahren werden bevorzugt Zeolithe des Pentasiltyps als Katalysatoren verwendet, die als Grundbaustein einen aus $SiO_4$-Tetraedern aufgebauten Fünfring gemeinsam haben. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen (s. Ullmanns Encyclopädie d. techn. Chem., 4. Aufl., Bd. 24, 1983). Diese Zeolithe können unterschiedliche chemische Zusammensetzung aufweisen. Es handelt sich hierbei um Alumino-, Boro-, Eisen-, Gallium-, Chrom-, Arsen- und Bismutsilikatzeolithe oder deren Gemische sowie um Alumino-, Boro-, Gallium- und Eisengermanatzeolithe oder deren Gemische. Insbesondere sind die isotaktischen Zeolithe zu nennen, die in der DE-OS 3o 06 471 beschrieben sind. Besonders gut geeignet sind die Alumino-, Boro- und Eisensilikatzeolithe des Pentasiltyps.

Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in 1,6-Hexandiamin- oder 1,3-Propandiamin- oder Triethylentetramin-Lösung mit und ohne Alkali- oder Erdalkalizusatz bei 100 bis 220°C unter autogenem Druck hergestellt. Die erhaltenen Aluminosilikatzeolithe enthalten je nach Wahl der Einsatzstoffmengen ein $SiO_2/Al_2O_3$-Verhältnis von 10 bis 40 000, insbesondere 30 bis 1 000. Auch lassen sich derartige Aluminosilikatzeolithe in etherischem Medium wie Diethylenglykoldimethylether, in alkoholischem Medium, wie Methanol bzw. 1,4-Butandiol, oder in Wasser synthetisieren.

Der Borosilikatkzeolith wird z.B. bei 90 bis 200°C unter autogenem Druck synthetisiert, indem man eine Borverbindung, z.B. $H_3BO_3$, mit einer Siliciumverbindung, vorzugsweise hochdispersem Siliciumdioxid, in wäßriger Aminlösung, insbesondere in 1,6-Hexandiamin- oder 1,3-Propandiamin- oder Triethylentetramin-Lösung mit und ohne Alkali- oder Erdalkalizusatz zur Reaktion bringt. Solche Borosilikatzeolithe können ebenfalls.hergestellt werden, wenn man die Reaktion statt in wäßriger Aminlösung in etherischer Lösung, z.B. Diethylenglykoldimethylether, oder in alkoholischer Lösung, z.B. 1,6-Hexandiol, durchführt.

Den Eisensilikatzeolith erhält man z.B. aus einer Eisenverbindung, vorzugsweise $Fe_2(SO_4)_3$, und einer Siliciumverbindung, vorzugsweise hochdispersem Siliciumdioxid, in wäßriger Aminlösung, insbesondere 1,6-Hexan diamin, mit und ohne Alkali- oder Erdalkalizusatz bei 100 bis 200°C unter autogenem Druck.

Die so hergestellten Alumino-, Boro- und Eisensilikatzeolithe können nach ihrer Isolierung, Trocknung bei 100 bis 160°C, vorzugsweise 110°C, und Calcinierung bei 450 bis 550°C, vorzugsweise 500°C, mit einem Bindemittel im Verhältnis 90:10 bis 40:60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:10, bevorzugt 75:25, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Preßlinge bei 110°C/16 Stunden getrocknet und bei 500°C/16 Stunden calciniert.

Man erhält auch vorteilhafte Katalysatoren, wenn der isolierte Alumino- bzw. Boro- bzw. Eisensilikatzeolith direkt nach der Trocknung verformt wird und erstmals nach der Verformung einer Calcinierung unterworfen wird. Die hergestellten Alumino-, Boro- und Eisensilikatzeolithe können aber auch in reiner Form, ohne Binder, als Stränge oder Tabletten eingesetzt werden. Diese Verformung erfolgt unter Zusatz von Verstrangshilfsbzw. Peptisierungsmittel, wie Hexaethylcellulose, Stearinsäure, Kartoffelstärke, Ameisensäure, Essigsäure, Oxalsäure, Salpetersäure, Ammoniak, Amine, Silicoester, Graphit oder deren Gemische.

Als Katalysatoren können auch Aluminosilikatzeolithe des Y-Typs verwendet werden, die aus Kieselsol (29 % $SiO_2$) und Natriumaluminat in wäßrigem Medium hergestellt werden. Diese Aluminosilikatzeolithe können vor ihrem Einsatz ebenfalls mit Bindern verformt werden. Die Zeolithe können auch als Mordenit-Typ vorliegen.

Diese unverformten oder verformten Zeolithe werden zur Gewinnung der erfindungsgemäß zu verwendenden Katalysatoren modifiziert, indem man sie mit Caesium dotiert oder silanisiert. Die Dotierung der Zeolithe kann durch Ionenaustausch oder durch Imprägnierung erfolgen. Praktisch stellt man die durch Dotierung mit Caesium modifizierten Kontakte z.B. so her, daß man den verformten Zeolithen in einem Steigrohr vorlegt und bei 20 bis 100°C z.B. die wäßrige Lösung eines Caesiumsalzes, wie eines Halogenids oder des Nitrats darüberleitet. Ein derartiger Ionenaustausch kann z.B. an der Wasserstoff-, Ammonium- und Na-Form des Zeolithen vorgenommen werden. Man kann die Aufbringung des Caesiums auf den Zeolithen z.B. auch so vornehmen, daß man das zeolithische Material z.B. mit einem Halogenid, dem Nitrat oder dem Oxid des Caesiums in wäßriger oder alkoholischer Lösung imprägniert. Sowohl an einen Ionenaustausch als auch an eine Imprägnierung schließt sich zumindest eine Trocknung z.B. bei Temperaturen von 100 bis 160°C und wahlweise eine abermalige Calcinierung, z.B. bei Temperaturen von 450 bis 550°C, an.

Der Ionenaustausch am Zeolithen kann z.B. auch so vorgenommen werden, daß man den Zeolithen in Strängen oder Pellets in einer Kolonne vorlegt und darüber z.B. eine wäßrige Caesiumcarbonat-Lösung bei leicht erhöhter Temperatur zwischen 30 und 80°C 15 bis 20 Stunden im Kreislauf leitet. Danach wird

der Katalysator mit Wasser ausgewaschen, z.B. bei ca. 150°C getrocknet und z.B. bei ca. 550°C calciniert.

Beim Imprägnieren verfährt man im einzelnen z.B. so, daß man Caesiumcarbonat in Wasser löst. Mit dieser Lösung wird dann der verstrangte oder unverstrangte Zeolith eine gewisse Zeit, ca. 30 min., getränkt. Die überstehende Lösung wird am Rotationsverdampfer von Wasser befreit. Danach wird der getränkte Zeolith z.B. bei ca. 150°C getrocknet und z.B. bei ca. 550°C calciniert. Dieser Tränkvorgang kann mehrmals hintereinander vorgenommen werden, um den gewünschten Metallgehalt einzustellen. Auch ist es möglich, z.B. eine Caesiumcarbonat-Lösung herzustellen und darin den reinen pulverförmigen Zeolithen bei 40 bis 100°C unter Rühren ca. 24 h aufzuschlämmen. Nach Abfiltrieren, Trocknen z.B. bei ca. 150°C und Calcinierung z.B. bei ca. 500°C kann das so gewonnene zeolithische Material mit und ohne Bindemittel zu Strängen oder Pellets oder Wirbelgut weiterverarbeitet werden.

Man stellt den Caesium-Gehalt der Zeolith-Katalysatoren auf 0,05 bis 3, vorzugsweise 0,1 bis 2 Gew.-% ein.

Die silanierten Zeolith-Katalysatoren werden durch Silanisierung von unverformten oder verformten Zeolithen gewonnen. Unter Silanisierung wird bekanntlich das Aufbringen von Siliciumverbindungen auf den festen Katalysator verstanden. Als Siliciumverbindung werden hierfür z.B. durch organische Reste oder Halogen substituierte Silane, Kieselsäureester oder Siliciumoxid verwendet. Verfahren der Silanisierung werden z.B. in den Deutschen Offenlegungsschriften 22 30 532, 26 17 571 und 32 31 498 und in der US-PS 3,980,586 beschrieben. Durch Silanisierung wird die Acidität des Zeolithen herabgesetzt.

Unter Silanisierung versteht man auch die Behandlung von Zeolithen mit z.B. $SiCl_4$, wodurch die dreiwertigen Elemente des Zeolithen wie z.B. Al gegen Silicium ersetzt werden (Beyer, H.K.; Bellnykaja, Y.: Studies in Surface Science and Catalysis 5, Catalysis by Zeolites, Amsterdam, Else. Sci. Publ. Comp. 1980, S. 203).

Beispielsweise verfährt man so, daß man das Zeolithpulver mit einer Siliciumverbindung, wie Tetramethylorthosilan in Wasser anmaischt, das Gemisch in einem Kneter homogenisiert und zu Strängen extrudiert. Danach werden die Stränge z.B. bei 100 bis 150°C getrocknet und z.B. bei 450 bis 550°C calciniert.

Die Silanisierung kann auch so vorgenommen werden, daß man den Zeolithen mit einem Siliciumhalogenid, wie $SiCl_4$ oder $(NH_4)_2SiF_6$ behandelt.

Hierbei wird unter $N_2$ entwässerter Zeolith in einem bei Raumtemperatur mit z.B. $SiCl_4$ gesättigten $N_2$-Strom behandelt unter langsamem Erwärmen des Reaktionsgefäßes mit 4°C/min auf 450 bis 550°C. Die Bildung von z.B. $AlCl_3$ wird beobachtet, d.h. Al wird durch Si im Zeolithgerüst ersetzt. Die Behandlung dauert bei 450°C bis 550°C ca. 2 Stunden. Durch die Substitution des Al mit Si wird die Anzahl der aciden Zentren im Zeolithen gesenkt. Der Dealuminierungsgrad hängt hauptsächlich von der Behandlungsdauer ab. Bei 400 bis 550°C während 2 Stunden wird das $SiO_2/Al_2O_3 = 2$ im Y-Zeolithen auf $SiO_2/Al_2O_3 = 40$ bis 100 erhöht.

Nach einer eventuell auftretenden Desaktivierung der zeolithischen Katalysatoren, die beim Verfahren der Erfindung durch Koksabscheidung eintreten könnte, lassen sich diese durch Abbrennen der Koksablagerung mit Luft oder mit einem Luft/$N_2$-Gemisch bei 400 bis 550°C, bevorzugt 500°C, in einfacher Weise regenerieren, wodurch sie ihre Anfangsaktivität zurückerhalten. Man kann auch durch eine partielle Verkokung (pre-coke) die Aktivität des Katalysators für ein Selektivitätsoptimum des gewünschten Reaktionsproduktes einstellen. Wird die Dehydratisierung in Gegenwart von inerten Gasen, wie Stickstoff ausgeführt, so kann damit die Produktzusammensetzung und Standzeit des Katalysators beeinflußt werden.

Im allgemeinen werden die Katalysatoren wahlweise als 2 bis 4 mm Stränge, als Tabletten mit 3 bis 5 mm Durchmesser, als Pulver mit Teilchengrößen von 0,3 bis 0,5 mm oder (als Wirbelkontakt) von 0,1 bis 0,5 mm eingesetzt.

Die Dehydratisierung der Aldehyde zu den Dienen führt man an den Zeolithen vorzugsweise bei Temperaturen von 150 bis 600°C, insbesondere bei 300 bis 500°C, durch. Die Belastung (WHSV) beträgt 0,1 bis 20, vorzugsweise 0,5 bis 5 g Aldehyd pro Gramm Katalysator und Stunde. Man kann auch in der Flüssigphase, z.B. bei Temperaturen von 30 bis 300°C dehydratisieren. Das Verfahren kann chargenweise oder kontinuierlich, drucklos oder unter Druck, z.B. in einem Strömungsreaktor, Rührkessel oder Wirbelreaktor, durchgeführt werden. Unumgesetzte Aldehyde können gegebenenfalls nach der Umsetzung destillativ von den entstandenen Dienen abgetrennt und erneut für die erfindungsgemäße Umsetzung verwendet werden.

## Beispiele 1 bis 6

Die Reaktionen mit 2-Methylbutanal als Einsatzstoff wurden wie folgt beschrieben durchgeführt.

Der Aldehyd wurde zum Zwecke der Dehydratisierung zum Dien unter isothermen Bedingungen in einen Rohrreaktor (Wendelform, Innendurchmesser 0,6 cm und Länge von 90 cm) eingeführt und in der Gasphase bei Temperaturen von 350 bis 450°C über einen erfindungsgemäßen Zeolith-Katalysator geleitet. Die erhaltenen Reaktionsprodukte wurden destillativ aufgearbeitet und durch Siedepunkt, Brechungsindex und NMR-Spektren charakterisiert. Die quantitative Bestimmung der REaktionsprodukte und der Ausgangsprodukte erfolgte gaschromatographisch. Die Art des Katalysators, die Temperatur, die Belastung (WHSV), der Umsatz und die Selektivität sind der folgenden Tabelle zu entnehmen:

Die Vergleichsbeispiele 5 und 6 sind ebenfalls in dieser Tabelle aufgenommen.

Tabelle

| Beispiel | 1 | 2 | 3 | 4 | 5 | 6[3)] |
|---|---|---|---|---|---|---|
| Katalysator | A | B | C | D | E | F |
| Temperatur | 400°C | 400°C | 400°C | 400°C | 400°C | 400°C |
| WHSV | 2 $h^{-1}$ | 2 $h^{-1}$ | 2 h-1 | 2 h-1 | 2 h-1 | 2 h-1 |
| Umsatz % | 51 | 38 | 33 | 52 | 37 | 35 |
| Selektivität % Isopren | 88 | 92 | 90 | 84 | 76 | 74 |
| Laufzeit[1)] | 120 h | 120 h | 220 h | 120 h | 48 h[2)] | 120 h |

1) Nach der angegebenen Laufzeit war noch keine Desaktivierung des Katalysators feststellbar.
2) zeigt nach dieser Zeit Desaktivierungserscheinungen
3) Werte nach 120 h Laufzeit

Katalysator A (mit Caesium dotierter Borosilikatzeolith)

Der Zeolith wurde in einer hydrothermalen Synthese aus 640 g $SiO_2$ (hochdisperse Kieselsäure), 122 g $H_3BO_3$, 8000 g einer wäßrigen Hexandiamin-Lösung (Mischung 50:50 Gew.-%) bei 170°C unter autogenem Druck in einem Rührautoklaven hergestellt. Nach Abfiltrieren und Auswaschen wurde das kristalline Reaktionsprodukt bei 100°C/24 Stunden calciniert. Es wurde ein Borosilikatzeolith vom Pentasiltyp erhalten, der 94,2 Gew.-% $SiO_2$ und 2,3 Gew.-% $B_2O_3$ enthielt. Aus diesem Zeolithen wurden 2 mm-Stränge hergestellt, die bei 100°C getrocknet und bei 500°C/24 Stunden calciniert wurden.
Diese Stränge wurden so mit einer wäßrigen Caesiumcarbonat-Lösung imprägniert, daß nach 2-stündigem Trocknen bis zu 130°C und anschließender 2-stündiger Calcinierung bei 540°C der Cs-Gehalt des Katalysators 0,4 Gew.-% betrug.

Katalysator B

Es wurde wie unter «Katalysator A» angegeben verfahren, wobei der Cs-Gehalt jedoch auf 0,75 Gew.-% eingestellt wurde.

Katalysator C

Es wurde wie unter «Katalysator A» angegeben verfahren, wobei der Cs-Gehalt jedoch auf 1 Gew.-% eingestellt wurde.

Katalysator D (Borosilikatzeolith-Katalysator, der silaniert ist)

100 g des nach dem ersten Absatz unter «Katalysator A» hergestellten Borosilikatzeolithen wurden mit 5 Gew.-% $Si(OCH_3)_4$ und 3 Gew.-% $NH_3$ in 130 g Wasser angemaischt. Das Gemisch wurde 45 min. in einem Kneter zu einer Masse verknetet und dann zu Strängen verformt. Die Stränge wurden bei 110°C getrocknet und bei 500°C/2 Stunden calciniert.

Vergleichskatalysatoren

Katalysator E (Borosilikatzeolith-Katalysator mit Rb dotiert)

Es wurde wie unter «Katalysator A» angegeben verfahren, wobei anstelle des Caesiumcarbonats nunmehr Rubidiumcarbonat eingesetzt wird und Rb-Gehalt auf 0,8 Gew.-% eingestellt wurde.

Katalysator F (Borosilikatzeolith-Katalysator ohne Dotierung und ohne Silanisierung)

Es wurde wie unter "Katalysator A" angegeben verfahren, wobei jedoch keine Dotierung oder Silanisierung des Zeolithen vorgenommen wurde.

## Patentansprüche

1. Verfahren zur Herstellung von Dienen durch Dehydratisierung von Aldehyden an dotierten Zeolith-Katalysatoren bei höheren Temperaturen, dadurch gekennzeichnet, daß man die Dehydratisierung unter Verwendung von Zeolith-Katalysatoren vornimmt, die mit Caesium dotiert oder die silaniert sind.

2. Verfahren nach Anspruch 1, dadurch gekennzeichnet, daß der mit Caesium dotierte Zeolith-Katalysator einen Cs-Gehalt von 0,05 bis 3 Gew.-% aufweist.

3. Verfahren nach Anspruch 1, dadurch gekennzeichnet, daß der Zeolith mit $Si(OCH_3)_4$ silaniert ist.

4. Verfahren nach Anspruch 1, dadurch gekennzeichnet, daß man als Aldehyde Verbindungen der Formel

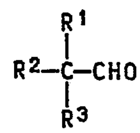 (I)

dehydratisiert, in der die Reste $R^1$, $R^2$ und $R^3$ gleich oder verschieden sind und jeweils einen aliphatischen, cycloaliphatischen, aromatischen oder heterocyclischen Rest mit 1 bis 10 C-Atomen bedeuten und $R^1$ und $R^2$ auch für Wasserstoffatome stehen können.

5. Verfahren nach Anspruch 1, dadurch gekennzeichnet, daß man als Aldehyde Verbindungen der Formel

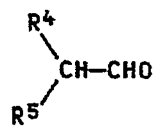 (II),

dehydratisiert, in der die Reste $R^4$ und $R^5$ gleich oder verschieden sind, $R^4$ einen Alkylrest mit 1 bis 3 C-Atomen oder ein Wasserstoffatom und $R^5$ einen Alkylrest mit 1 bis 8 C-Atomen oder einen Kohlenwasserstoffrest bedeutet, der zusammen mit $R^4$ einen Cyclohexylrest bildet.

6. Verfahren nach Anspruch 1, dadurch gekennzeichnet, daß man als Aldehyd 2-Methylbutanal dehydratisiert.

7. Verfahren nach Anspruch 1, dadurch gekennzeichnet, daß man Zeolithe vom Pentasil-Typ verwendet.

8. Verfahren nach Anspruch 1, dadurch gekennzeichnet, daß man einen Aluminosilikatzeolith verwendet.

9. Verfahren nach Anspruch 1, dadurch gekennzeichnet, daß man einen Borosilikatzeolith verwendet.

10. Verfahren nach Anspruch 1, dadurch gekennzeichnet, daß man einen Eisensilikatzeolith verwendet.

## Claims

1. A process for preparing a diene by dehydrating an aldehyde over a doped zeolite catalyst at elevated temperatures, which comprises performing the dehydration using a zeolite catalyst which has been doped with cesium or silanated.

2. A process as claimed in claim 1, wherein the cesium doped zeolite catalyst has a Cs content of from 0.05 to 3% by weight.

3. A process as claimed in claim 1, wherein the zeolite has been silanated with $Si(OCH_3)_4$.

4. A process as claimed in claim 1, wherein the aldehyde which is dehydrated is a compound of the formula

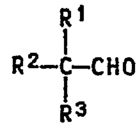 (I)

where $R^1$, $R^2$ and $R^3$ are identical to or different from one another and each is an aliphatic, cycloaliphatic, aromatic or heterocyclic radical of from 1 to 10 carbon atoms and $R^1$ and $R^2$ can each also be hydrogen atoms.

5. A process as claimed in claim 1, wherein the aldehyde which is dehydrated is a compound of the formula

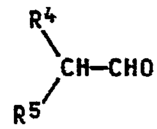 (II),

where $R^4$ and $R^5$ are identical to or different from one another, $R^4$ is alkyl of from 1 to 3 carbon atoms or hydrogen atom and $R^5$ is an alkyl radical of from 1 to 8 carbon atoms or a hydrocarbon radical which together with $R^4$ forms a cyclohexyl radical.

6. A process as claimed in claim 1, wherein the aldehyde which is dehydrated is 2-methylbutanal.

7. A process as claimed in claim 1, wherein a zeolite of the pentasil type is used.

8. A process as claimed in claim 1, wherein an aluminosilicate zeolite is used.

9. A process as claimed in claim 1, wherein a borosilicate zeolite is used.

10. A process as claimed in claim 1, wherein an iron silicate zeolite is used.

**Revendications**

1. Procédé de préparation de diènes par déshydratation d'aldéhydes sur des catalyseurs de zéolithe dopés, à températures élevées, caractérisé en ce qu'on effectue la déshydratation en utilisant des catalyseurs de zéolithe qui sont dopés au césium ou qui sont silanisés.

2. Procédé selon la revendication 1, caractérisé en ce que le catalyseur de zéolithe dopé au césium présente une teneur en Cs de 0,05 à 3% en poids.

3. Procédé selon la revendication 1, caractérisé en ce que la zéolithe est silanisée avec $Si(OCH_3)_4$.

4. Procédé selon la revendication 1, caractérisé en ce qu'on déshydrate en tant qu'aldéhydes des composés de formule

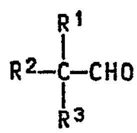 (I)

dans laquelle les restes $R^1$, $R^2$ et $R^3$ sont identiques ou différents et représentent chacun un reste aliphatique, cycloaliphatique, aromatique ou hétérocyclique de 1 à 10 atomes de carbone et $R^1$ et $R^2$ peuvent également représenter des atomes d'hydrogène.

5. Procédé selon la revendication 1, caractérisé en ce qu'on déshydrate en tant qu'aldéhydes des composés de formule

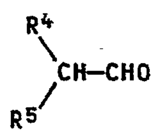 (II),

dans laquelle les restes $R^4$ et $R^5$ sont identiques ou différents, $R^4$ représente un reste alkyle de 1 à 3 atomes de carbone ou un atome d'hydrogène et $R^5$ un reste alkyle de 1 à 8 atomes de carbone ou un reste hydrocarboné qui forme avec $R^4$ un reste cyclohexyle.

6. Procédé selon la revendication 1, caractérisé en ce qu'on déshydrate en tant qu'aldéhyde du 2-méthylbutanal.

7. Procédé selon la revendication 1, caractérisé en ce qu'on utilise une zéolithe de type pentasil.

8. Procédé selon la revendication 1, caractérisé en ce qu'on utilise une zéolithe d'aluminosilicate.

9. Procédé selon la revendication 1, caractérisé en ce qu'on utilise une zéolithe de borosiliate.

10. Procédé selon la revendication 1, caractérisé en ce qu'on utilise une zéolithe de ferrosilicate.